# EUROPEAN PATENT APPLICATION

(11) **EP 3 678 142 A1**
(43) Date of publication of application: **08.07.2020**
(21) Application number: 19150374.7
(22) Date of filing: 04.01.2019
(51) Int. Cl.: G16H 20/00

(54) **DEVICE, SYSTEM AND METHOD FOR GENERATING A PERSONALIZED ACTION PLAN**

(71) Applicant: Koninklijke Philips N.V., 5656 AG Eindhoven (NL)
(72) Inventor: HUA, Qiao, 5656 AE Eindhoven (NL)
(74) Representative: de Haan, Poul Erik

(57) **Abstract**

The present invention relates to a device, system and method and computer program for generating a personalized action plan for a user. The device comprises a keyword extraction unit configured to obtain a text document comprising a treatment plan and to extract a plurality of keywords from said text document, a categorization data unit configured to collect categorization data from a data set, based on the extracted plurality of keywords, wherein data in said data set are categorized into different categories categorization data, and an action plan unit configured to generate the personalized action plan by combining the collected categorization data into action plan instructions.

## Description

### FIELD OF THE INVENTION

The present invention relates to a device, system and method for generating a personalized action plan.

### BACKGROUND OF THE INVENTION

In many countries, for example in China, most patients including pregnant women and children have only about three minutes time to talk to their doctors during a doctor's appointment. Moreover, doctors usually spend two thirds of their visiting time to read clinical data and analyze medical issues. Accordingly, doctors only have limited time to provide their patients with a comprehensive nutrition and lifestyle treatment plan.

In addition, most doctors lack sufficient training in the clinical nutrition domain. Hence, it is difficult for them to prescribe a personalized nutrition treatment plan. In addition many doctors lack sufficient knowledge about healthy and efficient training programs. Therefore, many patients do not know which exercises may be appropriate for them to loose weight and/or to generally improve their health and fitness.

However, there exist diverse programs from both public and private institutions to enhance the development of clinical nutrition in hospitals. For instance, Beijing government just released the policy that all maternal and child care hospitals in Beijing have to establish nutrition clinics in two years.

Still the majority of nutrition and lifestyle treatment plans prescribed by health care providers are too generic for patients or users to follow. Furthermore, many treatment plans comprise specialist terminology, in particular medical terminology, which makes these plans incomprehensible for patients. These facts lower the compliance of patients and users and influence the clinical outcomes in a negative way.

Moreover, some patients suffering from more than one disease or symptom may get more complicated treatment plans. For example, patients suffering from obesity, a high blood sugar level and rheumatism will probably get treatment plans that are not easily comprehensible and furthermore may not be easily applicable in their daily lives. Particularly patients concerned with such complications may therefore feel confused and stressed when facing their prescribed treatment plans.

### SUMMARY OF THE INVENTION

It is an object of the present invention to generate a personalized action plan for a user in order to improve the user's compliance with a treatment plan provided by a doctor or therapist and/or that increases or even maximizes effectiveness with regard to the original treatment plan.

In a first aspect of the present invention a device for generating a personalized action plan for a user is presented, the device comprising:
- a keyword extraction unit configured to obtain a text document comprising a treatment plan and to extract a plurality of keywords from said text document,
- a categorization data unit configured to collect categorization data from a data set, based on the extracted plurality of keywords, wherein data in said data set are categorized into different categories, and
- an action plan unit configured to generate the personalized action plan by combining the collected categorization data into action plan instructions.

In a second aspect of the present invention a system for generating a personalized action plan for a user is presented, the system comprising:
- a device for generating a personalized action plan for a user as disclosed herein, and
- a data input unit configured to generate input data, and/or
- a data output unit configured to output the personalized action plan.

In yet further aspects of the present invention, there are provided a corresponding method and computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to convert an original treatment plan as prescribed by a doctor in easily comprehensible and practicable instructions to patients (i.e. users) to support them in implementing their treatment plan.

After obtaining a text document, i.e. text data, comprising a treatment plan, prescribed e.g. by a doctor, a plurality of keywords are extracted by the keyword extraction unit. Keywords extraction may be performed according to a predefined scheme, for example, wherein keywords are distinguished from irrelevant words in the text document. According to the predefined scheme only terms related to predefined (thematic) groups may be selected, for example, wherein the predefined (thematic) groups may comprise at least one of a food related group, a sports related group, a shopping related group etc. There may be extracted any of a noun, a verb, an adjective and an adverb as well as a corresponding numeral and/or quantifier. For example, there may be extracted nouns, verbs and adjectives related to food such as "vegetables", "eat", "fatty" and the like. Conjunctions, pronouns and/or prepositions may be excluded from extraction, however.

As an example, provided the original treatment plan reading "reduce intake of high fat food", the keywords extracted may be "reduce", "high", "intake", "fat", and "food" if it is defined that all nouns, verbs and adjectives are subject to extraction.

The categorization data unit is configured to collect the categorization data from a data set based on the extracted plurality of keywords. The data in the data set may be categorized into different categories and pre-stored in a database.

Given the collected categorization data the action plan unit is configured to generate the personalized action plan. This may be achieved by combining the collected categorization data into action plan instructions. Combination of the categorization data may be supported by (predefined) action plan templates which are configured to define a language structure in order to get grammatically correct instructions from the action plan unit. The action plan templates may follow a predefined sentence structure with a predefined sequence of word classes. In particular, it may be examined to which word class categorization data belong to. Accordingly, at least a noun and a verb are combined to generate a meaningful action plan instruction whereas an instruction comprising only adjectives will not be generated.

The database comprising prefabricated action plan templates may either be stored in the device itself, particularly in the action plan unit, or may be stored externally. Combination and conversion of the collected categorization data may be supported by the access to said database. The action plan generated by the action plan unit may then be in accordance with the prefabricated templates.

Apart from this, it is preferred that the action plan unit is configured to generate action plan instructions that comprise short sentences which are easy to understand and particularly avoid specialist terminology. In fact, the action plan unit may be configured to generate a plurality of instructions which help the user to implement his/her treatment plan step by step.

Advantageously, the categorization data in the same subcategory include both a general term of a category and at least one specific term in said category. For example, the categorization data may comprise "fat food" as general term and "beef' and "pork" as specific terms in the same category. As a result, the collected categorization data may include both a general term and a specific term. This ensures that at least some of the action plan instructions are specific enough to be actionable.

Advantageously, the device for generating the personalized action plan may be implemented in any of a computer, tablet, smartphone, smartwatch or similar device, wherein the device for generating the personalized action plan preferably has access to a database comprising prefabricated action plan templates.

The present invention overcomes the aforementioned current problems by converting prescribed treatment plans into comprehensible action plan instructions. The solution proposed helps the user prioritize and sequence his/her treatment. Hence, users may follow their treatment plan in an easier and more actionable manner. This is further supported by providing concise instructions which help the user understand quickly what is required of him/her. As there may be provided a variety of concise action plan instructions in support of the original treatment plan, the user can choose those instructions which fit best into his/her daily routine. Accordingly, the compliance with a treatment plan as prescribed can be increased by the present invention. The same applies to the effectiveness of the original treatment plan. Therefore, the user can achieve the target of the original treatment plan in a short period of time (e.g. a single week) resulting in an improved health, well-being and/or fitness. Another advantage of the present invention is that health care providers can be relieved from unnecessary subsequent visits by their patients.

In an embodiment, the device for generating a personalized action plan for a user further comprises a user data input configured to obtain user data, wherein the user data may comprise at least one of age, gender, weight, lifestyle data, fitness data, medical data, exercise behaviour data and dietary behaviour data.

The user data input may be a wired or wireless data interface configured retrieve or receive the respective data.

Lifestyle data may comprise information about at least one of personal preferences, e.g. with respect to food, eating habits, exercises and/or shopping, working hours, leisure activities, and appointments.

Fitness data may comprise information about the user's fitness and may particularly comprise information about the user's fitness history and/or a fitness indicator such as VO2max data, i.e. data indicating the maximum rate of oxygen consumption a person can achieve during sustained physical activity and/or metabolic equivalent data.

Medical data may comprise information about the user's state of health. In particular, said data may comprise the user's medical history, blood pressure, heart rate, blood glucose level, injuries and/or diseases.

Exercise behaviour data may comprise information about the exercise behaviour of the user. In particular, this data may comprise information about the times, frequency and/or duration the user does sports. Furthermore, information about the type of exercises may be provided, for example, whether the user goes swimming or does press-ups.

Dietary behaviour data may comprise information about the user's dietary behaviour. For example, dietary behaviour data comprises information about the user's eating times and kind of food (comprising beverages) he/she eats or drinks.

In another embodiment, the device comprises a matching unit configured to match an extracted keyword to categorization data, including the extracted keyword, in the data set, wherein the categorization data unit is configured to collect the categorization data that are in the same category or subcategory as the matched categorization data.

Preferably, the matching unit is configured to match each extracted keyword to the categorization data. More preferably, the matching unit is configured match the keywords to the categorization data starting from a lowest level of a subcategory of the categorization data. In this embodiment, the categorization data unit is configured to collect the categorization data from the data set based on the matched categorization data.

In another embodiment, the device for generating a personalized action plan further comprises a filtering unit configured to filter the action plan instructions, particularly according to the user data.

Filtering the action plan instructions means that particular instructions are dropped whereas other instructions are kept. The filtering may be performed according to a predefined scheme, wherein said scheme may take into account the content of the instructions, the kind and/or number of words the instructions are formulated with, and/or the length of the instructions.

In particular, irrelevant instructions may be filtered out, wherein the relevance of instructions may comply with a predefined scheme. For example, instructions comprising a number of words above a predefined threshold may be filtered out. Likewise, instructions comprising long and/or complicated terms may be subject to filtering. By reducing the number (of particularly) complicated instructions the user is capable to quickly understand what to do in order to comply with the treatment plan provided by his/her doctor.

Furthermore, if the instructions are filtered according to their (semantic) content the effectiveness of the treatment plan may be enhanced. For example, instructions regarding physical activity may be filtered out, whereas instructions regarding dietary behaviour may be kept.

Preferably, the filtering unit is configured to filter the action plan instructions based on the user data provided by the user data input. If the user data indicate, for example, that the user prefers doing exercises rather than watching his/her diet, instructions related to dietary behaviour may be filtered out in order to enhance the user's compliance with the treatment plan. Likewise, the user data comprising the user's state of health may indicate which action plan instructions are of higher relevance than others.

In a preferred embodiment, the device further comprises a prioritizing unit configured to prioritize the collected categorization data and/or to prioritize the action plan instructions based on the user data. Prioritizing the collected categorization data may be based on the keywords.

In particular, the collected categorization data may be prioritized according to the number of keywords matching the categorization data. For example, given the keywords "high", "fat" and "food" and the categorization data "fat", "fat food" and "high fat food", the categorization data "high fat food" may be prioritized since said categorization data comprises the highest number of keywords. Preferably, the collected categorization data are prioritized prior to generating the personalized action plan.

Prioritizing the action plan instructions may be performed according to a predefined scheme, wherein said scheme may take into account the content of the instructions, the kind and/or number of words the instructions are formulated with, and/or the length of the instructions. For example, the instructions may be prioritized according to a scheme indicating the importance of the instructions with respect to the user's health and/or fitness. The instructions may also be ordered according to their length with the shortest instruction beginning. This has the advantage that the user may quickly understand what to do in order to comply with the treatment plan. Likewise, instructions regarding physical activity may be prioritized over instructions regarding dietary behaviour and/or food shopping behaviour, for example.

Furthermore, the instructions may be prioritized according to the personal preferences of the user and the user's working hours. In particular, prioritizing may be performed in accordance with the user's medical data. For instance, for an overweight pregnant woman with gestational diabetes it is of higher priority to keep her blood glucose level lower than a predefined threshold than to keep her weight below a particular threshold. In case both conditions, i.e. the woman's overweight and her blood sugar level, represent an equal health risk, prioritizing may be in accordance with the woman's treatment plan compliance, for example.

In an advantageous embodiment, the device for generating the personalized action plan further comprises an effectiveness unit configured to generate effectiveness data based on the user data and the personalized action plan, wherein the effectiveness data comprises information about an effectiveness of the personalized action plan, wherein the action plan unit is configured to adapt the personalized action plan based on the effectiveness data.

In the effectiveness unit the action plan instructions are compared to the user data. For example, instructions related exercise behaviour may be compared with the fitness data and/or medical data. If fitness data show a great improvement in the user's fitness whilst the personalized action plan comprises instructions to do exercises, for example, the effectiveness unit may assume that the effectiveness of the personalized action plan is high. Likewise, the dietary behaviour instructions may be compared with the medical data to check whether the instructions have led to any improvement in the user's health. Comparison of both data may be performed using a rating scheme, for example. The effectiveness data generated may be processed by the action plan unit to adapt the personalized action plan. If the effectiveness of instructions related to exercises turns out to be higher than the effectiveness of instructions related to dietary habits, for example, the actions plan unit may highlight exercise instructions. At the same time, the action plan unit may select only categorization data related to exercises for generating the personalized action plan.

In a further embodiment, the device further comprises a compliance unit configured to generate compliance data based on the user data and the personalized action plan, wherein the compliance data comprises information about a compliance of the user with the personalized action plan, wherein the action plan unit is configured to adapt the personalized action plan based on the compliance data.

In the compliance unit the action plan instructions are compared to the actual behaviour of the user as indicated by the user data. In particular, instructions related exercise behaviour may be compared with the exercise behaviour data. Likewise, dietary behaviour instructions may be compared with the dietary behaviour data. The better the user's actual behaviour fits to the personalized action plan generated, the higher will be the compliance with the personalized action plan. The compliance data is configured to represent the compliance with the personalized actions plan.

There are also embodiments conceivable, in which the effectiveness unit further uses the compliance data to generate the effectiveness data. In fact, a higher compliance may be accompanied by a higher effectiveness of the instructions.

In yet another embodiment of the device, the prioritizing unit is configured to prioritize the action plan instructions according to the effectiveness data and/or the compliance data.

For example, the compliance data may show that the user's compliance with action plan instructions regarding exercise behaviour is high, whereas the user's compliance with dietary behaviour instructions are low. In this case the prioritizing unit may prioritize exercise behaviour instructions over dietary behaviour instructions. This way, the effectiveness of the personalized action plan can be increased. However, there also may be situations where the user's compliance with the instructions given is high concerning both exercise and dietary behaviour, but the effectiveness of the exercise instructions turns out to be higher than the effectiveness of the dietary behaviour instructions. In this case the prioritizing unit may prioritize instructions with respect to exercise behaviour.

In another embodiment, the filtering unit of the device is configured to filter the action plan instructions according to the effectiveness data and/or the compliance data.

In particular, action plan instructions showing little effectiveness or compliance may be subject to filtering. In fact, the effectiveness unit may determine an effectiveness level and the filtering unit may filter out instructions corresponding to an effectiveness level below a predefined threshold. The same applies to the compliance unit, i.e. the compliance data, respectively.

In an advantageous embodiment, the categorization data are categorized into different categories comprising physiological target, food type and action type. Categorization data may be divided into only said three categories, but may likewise be categorized in further categories.

The category physiological target may comprise information about input and output of energy and nutrients. The category food type may comprise information about types and amount of food (comprising beverages) and the category action type may comprise information about actions related to food consumption (comprising actions related to eating food and shopping food) and physical activities.

In still another embodiment, the device further comprises a text generation unit configured to generate a text document from input data, wherein the input data comprise an image and/or a voice recording, and/or the device further comprises a data output unit configured to output the personalized action plan, wherein the data output unit comprises any of a display, a printer and a loudspeaker, and/or the device further comprises a data input unit configured to generate the input data, wherein the data input unit comprises any of a camera, a scanner and a voice recorder.

Hence, the personalized action plan may be presented to the user via a data output unit comprised in his/her smartphone, smartwatch, television set etc. Accordingly, the action plan may be displayed via a display and/or communicated via a loudspeaker and/or printed by a printer.

The data input unit, the data output unit and the text generation unit may be wired or wireless data interfaces, e.g. Bluetooth interfaces, WiFi interfaces, USB interfaces, computer network interfaces, mobile communication interfaces, etc., for obtaining (i.e. retrieving or receiving) the respective data. In particular, the data input unit may be represented by a camera, a voice recorder, a smartphone and the like.

In a preferred embodiment, the action plan unit has access to one or more action plan templates. Said templates may be stored in the action plan unit itself, in another unit of the device or externally. Advantageously, the templates may be classified into one or more predefined template classifications. In particular, the templates may be classified into the template classifications "what to eat?", "how to prepare?" and "what to do?", wherein the template classification "what to eat?" may comprise action plan templates that support generating action plan instructions, for example "reduce eating cake" and "reduce eating sausage" etc. In fact, the templates may be predefined, preferably according to a predefined scheme. For example, a template may comprise an imperative, a quantifier and a noun. According to categorization data characteristics, the collected categorization data or prioritized collected categorization data will be filled in the action plan templates.

In a further embodiment of the device for generating a personalized action plan, the action plan unit is configured to supplement at least one action template from each predefined template classification.

In an embodiment of the system for generating a personalized action plan, the system comprises a text generation unit configured to generate a text document from input data, wherein the input data comprise an image and/or a voice recording. In general, however, the input data may already comprise a text document.

In another embodiment of the system, the data input unit may comprise any of a camera, a scanner and a voice recorder.

In a further embodiment of the system, the data output unit may comprise at least one of display, a printer and a loudspeaker.

In an embodiment of the method for generating a personalized action plan, the method further comprises matching an extracted keyword to categorization data, including the extracted keyword, in the data set, and collecting categorization data that are in the same category or subcategory as the matched categorization data.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a device for generating a personalized action plan according to the present invention,
Fig. 2 shows a schematic diagram of a second embodiment of a device for generating a personalized action plan according to the present invention,
Fig. 3 shows a schematic diagram of a third embodiment of a device for generating a personalized action plan according to the present invention,
Fig. 4 shows a schematic diagram of a fourth embodiment of the device for generating a personalized action plan according to the present invention,
Fig. 5 shows an exemplary implementation of an embodiment of a device for generating a personalized action plan according to the present invention,
Fig. 6 shows an exemplary categorization of categorization data,
Fig. 7 shows a flow chart of a first embodiment of a method for generating a personalized action plan for a user according to the present invention,
Fig. 8 shows a flow chart of a second embodiment of a method for generating a personalized action plan for a user according to the present invention, and
Fig. 9 shows a schematic diagram of an embodiment of a system for generating a personalized action plan according to the present invention.

### DETAILED DESCRIPTION OF THE INVENTION

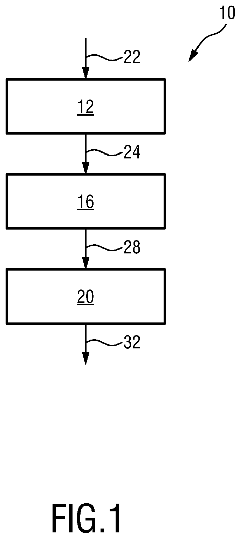

Fig. 1 shows a schematic diagram of a first embodiment of the device 10 for generating a personalized action plan according to the present invention. The device 10 comprises a keyword extraction unit 12, a categorization data unit 16 and an action plan unit 20.

In this embodiment, the keyword extraction unit 12 is configured to obtain a text document 22 comprising a treatment plan. From said text document 22 the keyword extraction unit 12 is configured to extract a plurality of keywords 24. Using the keywords 24 the categorization data unit 16 collects categorization data, wherein the categorization data are categorized into different categories according to a predefined structure. In the action plan unit 20 the collected categorization data 28 are combined into action plan instructions, i.e. the personalized action plan 32, based on one or more templates.

Preferably and optionally, the device further comprises a matching unit 14 configured to match an extracted keyword 24 to the categorization data including the extracted keyword. The categorization data unit is configured to collect the matched categorization data 26 that are in the same category or subcategory as the matched categorization data, resulting in collected categorization data 28.

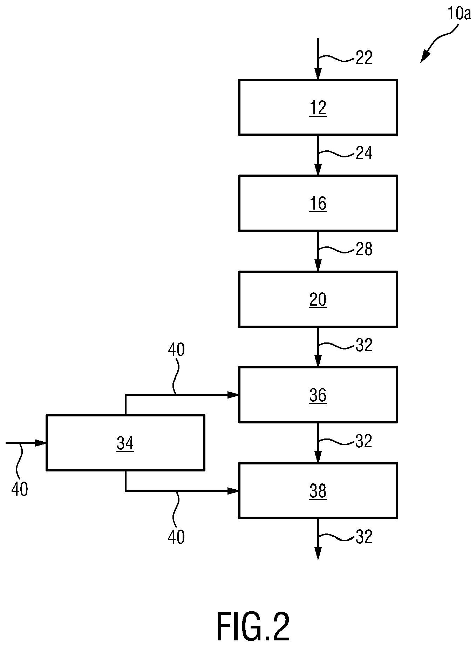

Fig. 2 shows a schematic diagram of a second embodiment of the device 10a for generating a personalized action plan according to the present invention. The device 10a comprises a keyword extraction unit 12, a categorization data unit 16, an action plan unit 20, a user data input 34, a filtering unit 36 and a prioritizing unit 38.

The action plan unit 20 of this embodiment comprises a database comprising templates to generate the personalized action plan 32 including a plurality of action plan instructions.

Furthermore, in this embodiment the user data input 34 is configured to obtain user data 40, which are used both by the filtering unit 36 and the prioritizing unit 38 to adapt the personalized action plan 32. In particular, the user data 40 comprises lifestyle data, exercise behavior data and dietary behavior data. Exercise behavior data comprises information about the frequency the user plays tennis and goes swimming, for example, whereas dietary behavior data may comprise information about the user's eating habits in particular with respect to fatty and sweet food. The lifestyle data comprises information about the user's preferences with respect to shopping food, for example.

Taking this information provided by the user data 40 into account the filtering unit 36 filters particular action plan instructions. For example, in case the lifestyle data reveal that the user prefers shopping fatty food rather than sweets, the filtering unit 36 may particularly filter out all action plan instructions regarding sweets since the effect of said instructions would be little anyway. In fact, this is in accordance with the dietary behavior data. Concerning the exercise behavior, the filtering unit 36 may filter out more instructions related to tennis than to swimming if the exercise behavior data reveal that the user goes swimming more frequently than playing tennis.

In the prioritizing unit 38 the filtered action plan instructions 32 are prioritized according to the user data 40. In this embodiment, the instructions are prioritized particularly according to the lifestyle data. In case the lifestyle data comprise information that the user prefers doing sports over taking care of his/her diet, the prioritizing unit 38 will prioritize instructions related to sports over instructions related to dietary behavior assuming that the user's compliance with instructions prioritized in this way is higher.

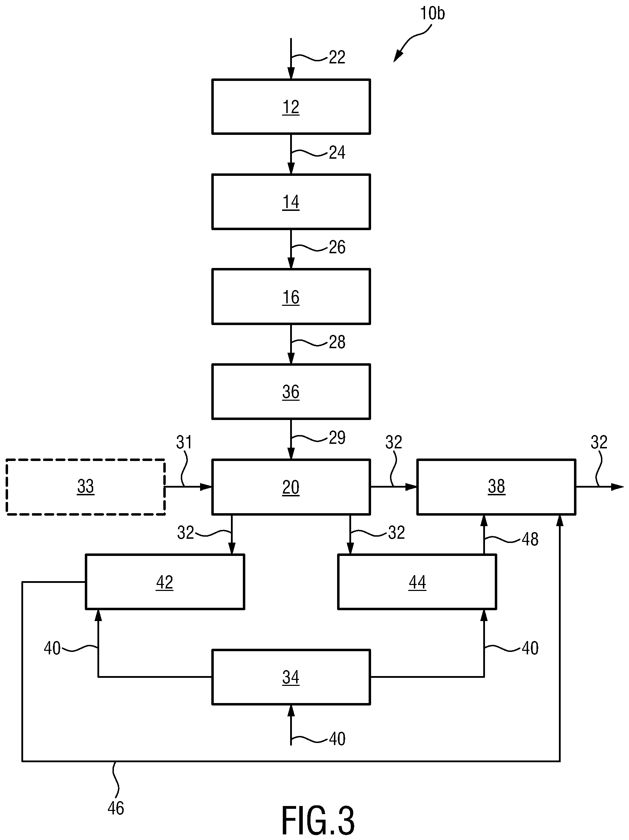

Fig. 3 shows a schematic diagram of a third embodiment of the device 10b for generating a personalized action plan according to the present invention. The device 10b comprises a keyword extraction unit 12, a matching unit 14, a categorization data unit 16, a filtering unit 36, an action plan unit 20, a user data input 34, an effectiveness unit 42, a compliance unit 44 and a prioritizing unit 38. Optionally, the device 10b may comprise a database unit 33.

In this embodiment, the keyword extraction unit 12 is configured to obtain a text document 22 comprising an original treatment plan. From the text document 22 keywords 24 are extracted according to a predefined scheme. In this embodiment, nouns, verbs and adjectives related to food, sports shopping and/or lifestyle are extracted from the text document 22. The matching unit 14 is configured to match the extracted keywords 24 to the categorization data which includes the keywords. Using the matched categorization data 26, the categorization data unit 16 is configured to collect all categorization data that are in the same subcategory from the data set, resulting in collected categorization data 28. The categorization data are pre-categorized into different categories and subcategories according to a predefined structure in the data set.

The user data input 34 may be configured to obtain user data 40, which are used both by the effectiveness unit 42 and the compliance unit 44. The user data 40 may comprise lifestyle data, exercise behavior data and dietary behavior data, for example. Furthermore, the user data input 34 is configured to obtain the action plan 32.

In the effectiveness unit 42 the user data 40 and the action plan 32 comprising action plan instructions are compared with each other. The effectiveness unit 42 is configured to generate effectiveness data 46 from said comparison comprising information about the effectiveness of an action plan instruction for the user. For example, if the personalized action plan 32 provided to the user comprised the instruction to go for a walk twice a day, but the user never went for a walk according to the user data 40, the effectiveness unit 42 is configured to indicate that instructions related to walking are not effective. Accordingly, the effectiveness unit 42 is configured to generate effectiveness data 46 comprising information that action plan instructions related to walking are not effective for the user. On the other hand, if the user complied with said instructions, the effectiveness unit 42 may be configured to generate effectiveness data 46 comprising information that the effectiveness is high.

Apart from the effectiveness unit 42 the compliance unit 44 is configured to obtain the user data 40 and the personalized action plan 32. In the compliance unit 44 the user data 40 and the personalized action plan 32, i.e. the one or more action plan instructions, are compared with respect to a compliance of the user with the instructions given to him/her. In particular, the compliance unit 44 is configured to generate compliance data 48 comprising information about the user's compliance with the action plan instructions.

The prioritizing unit 38 is configured to obtain the effectiveness data 46 and the compliance data 48. Taking the effectiveness data 46 and the compliance data 48 into account, the prioritizing unit 38 is configured to prioritize the action plan instructions comprised in the personalized action plan 32. In this embodiment, the higher the effectiveness of an action plan instruction and the higher a compliance of the user with an actions plan instruction, the higher the priority assigned to an action plan instruction. The prioritizing unit 38 then provides a personalized action plan 32 with prioritized action plan instructions.

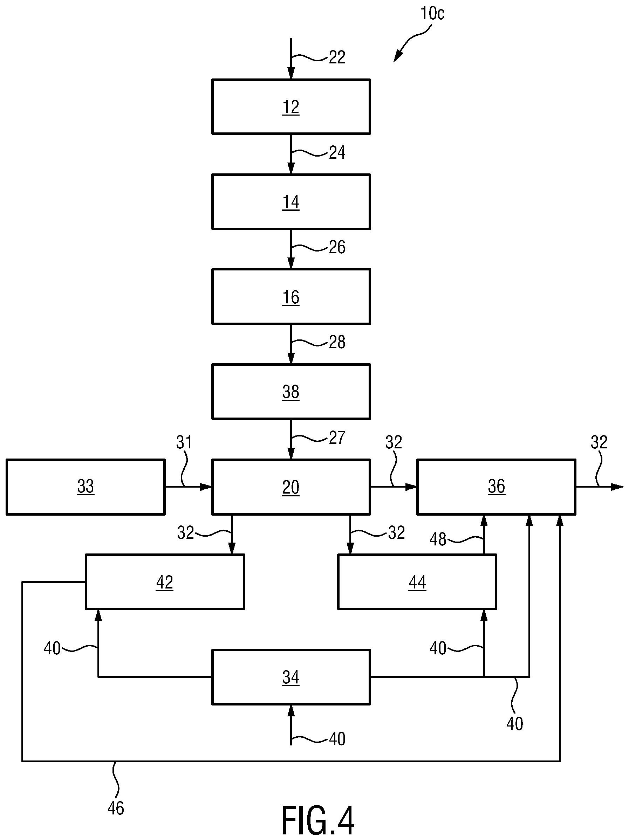

Fig. 4 shows a schematic diagram of a fourth embodiment of the device 10c for generating a personalized action plan according to the present invention. The device 10c comprises a keyword extraction unit 12, a matching unit 14, a categorization data unit 16, a prioritizing unit 38, an action plan unit 20, a database unit 33, a user data input 34, an effectiveness unit 42, a compliance unit 44 and a filtering unit 36.

From a text document 22 obtained the keyword extraction unit 12 is configured to extract keywords 24 according to a predefined scheme, wherein all nouns, verbs, numbers, adjectives and adverbs are extracted. For example, for an original treatment plan reading "reduce intake of high fat food", the nouns "intake" and "food", the verb "reduce" and the adjectives "high" and "fat" are extracted. The matching unit 14 is configured to match the extracted keywords 24 to categorization data including said keywords. Based on the matched categorization data 26 the categorization data unit 16 is configured to collect categorization data, wherein the categorization data are pre-categorized into different categories/subcategories according to a predefined structure. In particular, there are collected all categorization data which coincide with or include the keywords and all categorization data which are in the same sub-categories with the matched categorization data. Accordingly, there may be collected the categorization data "fat" and "fat food", for example.

In the prioritizing unit 38 the collected categorization data 28 may be prioritized, resulting in collected and prioritized categorization data 27. In particular, the collected categorization data 28 may be prioritized according to the keywords 24. More particular, the better categorization data match with keywords, the higher their priority assigned by the categorization data unit. In fact, also the categorization data belonging to the same subcategory of the prioritized matched categorization data may be prioritized.

In the action plan unit 20 the collected and prioritized categorization data 27 are combined into action plan instructions, i.e. the personalized action plan 32. This is achieved by supplementing (prefabricated) action plan templates, from a database unit 33 with the collected and prioritized categorization data 27. In particular, the collected and prioritized categorization data may be filled in the templates. Furthermore, the templates may be pre-classified in different classifications and the action plan unit 20 may be configured to supplement at least one action plan template from each template classification. Accordingly, there may be generated at least one action plan instruction corresponding to each classification by the action plan unit 20.

The user data input 34 may be configured to obtain user data 40, which are used both by the effectiveness unit 42 and the compliance unit 44, wherein the effectiveness unit 42 is configured to generate effectiveness data 46 and wherein the compliance unit 44 is configured to generate compliance data 48.

Both the effectiveness data 46 and the compliance data 48 may be used by the filtering unit 36 to filter the action plan instructions 32. However, the filtering unit 36 may likewise be configured to filter out all action plan instructions except for at least one from each classification. For example, the filtering unit may select a predefined number of action plan instructions, wherein the templates the instructions are based on are each in a different category. Apart from that the filtering unit 36 may be configured to filter the action plan instruction in accordance with the user data 40. For example, the user data 40 may comprise data with respect to a health status of the user. Therefore, for a user with a broken leg, action plan instructions with respect to sports plans are filtered out for said user.

Optionally, prioritizing unit 38 of the device 10c may be configured to obtain any of the effectiveness data 46, the compliance data 48 and the user data 40. In fact, the prioritizing unit 38 may be configured to prioritize the action plan instructions based on any of said data.

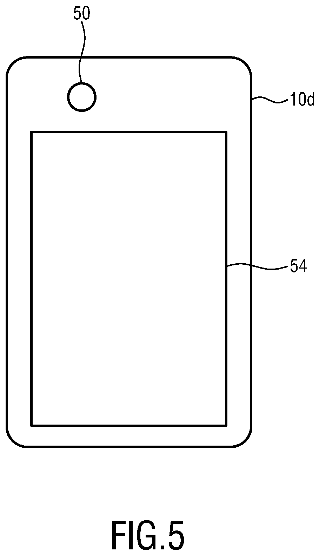

Fig. 5 shows a first exemplary implementation of an embodiment of a device for generating a personalized action plan according to the present invention. In this embodiment the device 10d is implemented in a smartphone, wherein the smartphone comprises a keyword extraction unit 12, a categorization data unit 16, and an action plan unit 20. The smartphone further comprises a data input unit 50 in the form of a camera, a text generation unit 52 and a data output 54 being implemented as a display. The camera is configured to photograph a treatment plan. The corresponding image data, i.e. input data 56, are processed by the text generation unit 52 in order to generate a text document comprising said treatment plan. Subsequently keywords are extracted from the text document for generating a personalized action plan 32 for the user. Once the personalized action plan 32 has been generated by the action plan unit 20, the plan is displayed on the smartphone's display.

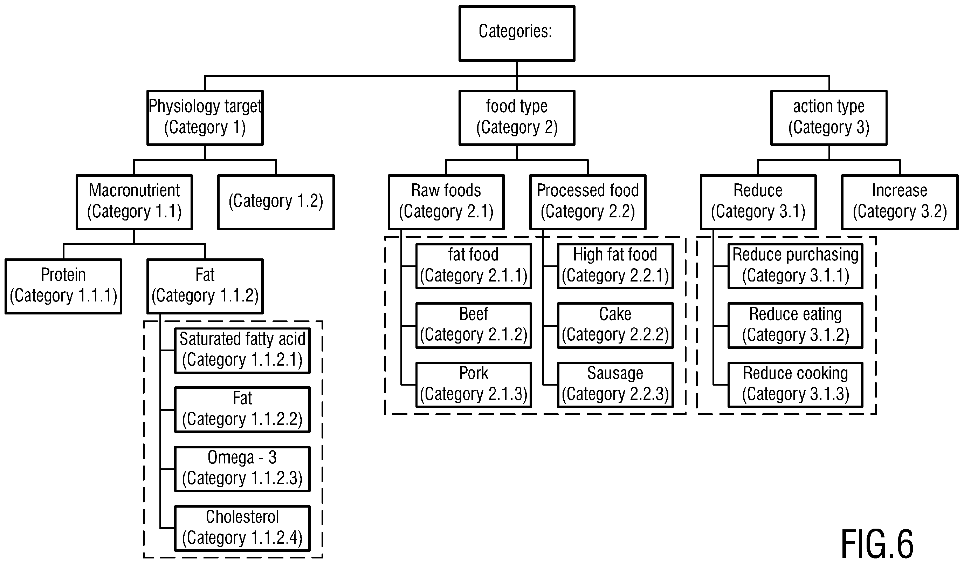

Fig. 6 shows an exemplary categorization of categorization data. In this exemplary categorization the categorization data are categorized in three categories, wherein "Category 1" corresponds to categorization data related to "physiology target", "Category 2" corresponds to categorization data related to "food type" and "Category 3" corresponds to categorization data related to "action type". The categorization data may further be categorized in sub-categories. For example, the categorization data "macronutrient" under "Category 1" may itself represent a sub-category comprising the further sub-categories "protein" and "fat", wherein "fat" may comprise further sub-categories saturated fatty acid, fat, Omega-3 and Cholesterol.

The categorization data encircled in Fig. 6 have been matched and selected in accordance with the keywords "reduce", "intake", "high", "fat" and "food" originating from an original treatment plan reading "reduce intake of high fat food". From Fig. 6, it can be understood that the matching is started from the lowest level of a sub-category. When the categorization data is matched to a certain sub-category, all the categorization data belonging to a same sub-category are collected. For example, the categorization data "Fat" (encircled in Fig. 6) under the sub-category "Fat" of "Macronutrient" in Category 1 is matched according to the keywords "fat", and then the categorization data "saturated fatty acid", "omega 3" and "cholesterol" belong to the same sub-category as the matched categorization data "fat" are collected. All these collected categorization data related to the keyword "fat" are collected as shown in Fig. 6 by a dashed box.

In the same way, the categorization data related to "food", "high" and "reduce" can be collected (shown in Fig. 6 with dashed boxes).

As there is no categorization data comprising "intake", the keyword "intake" is discarded in this example.

In the example, all the categorization data collected include all data in the dashed boxes. i.e. the collected categorization data include: "Saturated fatty acid", "Fat", "Omega-3", "Cholesterol", "Fat food", "Beef, Pork", "High fat food", "Cake, Sausage", "Reduce purchasing", "Reduce eating", "Reduce cooking".

With respect to a prioritization of the categorization data collected, the prioritizing unit 38 may be configured to prioritize the categorization data that match the keywords best. In fact, those categorization data collected may be prioritized which match best to the keywords. In other words, the categorization data comprising the highest number of words coinciding with keywords is selected and said data plus the other categorization data in the same (sub)category are prioritized for generating the personalized action plan. In the example of Fig.6, the categorization data "High fat food" has the most number of words matches the keywords (matches three keywords), therefore, the categorization data in the sub-category of "High fat food" will be prioritized, i.e the categorization data "High fat food", "Cake" and "Sausage" will be prioritized.

When categorization data are collected, they are filled in templates to generate the action plan instructions. The action plan templates may be classified in three template classifications comprising "what to eat?", "how to prepare?" and "what to do?", for example. In the classification "what to eat?" the templates are constructed according to a scheme wherein an imperative is followed by a gerund and a noun. In the classification "how to prepare?" the templates are constructed by an imperative followed by a noun followed by the preposition "for" and a quantifier along with a unit for said quantifier. In the classification "what to do?" the templates are structured in accordance with an imperative followed by a noun followed by a quantifier followed by an adverb and a time unit. With respect to Fig. 6 the categorization data collected by the action plan unit 20 are "reduce purchasing", "reduce eating", "reduce cooking", "cake", "sausage", "beef', "pork", "saturated fatty acid", "omega 3" and "cholesterol". Accordingly, said data may be used to fill in the action plan templates mentioned.

For example, the action plan template of the classification "what to eat?" may be a "verb verb noun"-structure, particularly a "imperative gerund noun"-structure, which when filled with said collected categorization data may lead to a generation of the following action plan instructions: "Reduce eating cake"; "Reduce eating sausage"; "Reduce cooking cake"; "Reduce cooking sausage"; "Reduce purchasing cake"; "Reduce purchasing sausage".

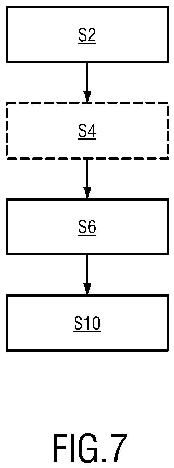

Fig. 7 shows a flow chart of an embodiment of a method for generating a personalized action plan for a user according to the present invention.

In a first step S2 keywords are extracted from a text document. In a second step S6 categorization data are collected with respect to the extracted keywords 24. To collect the categorization data, the method of this embodiment may comprise a further step S4 comprising matching the extracted keywords to categorization data including the keywords in a data set, wherein the data set comprises a plurality of categorization data that are categorized into different categories. Preferably, the matching starts from the lowest level of the sub-categories, and then there are collected all the categorization data which are in the same sub-categories as the matched categorization data.

Based on the collected categorization data 28, the personalized action plan 32 is generated in step S10 by combining the collected categorization data 28 into action plan instructions.

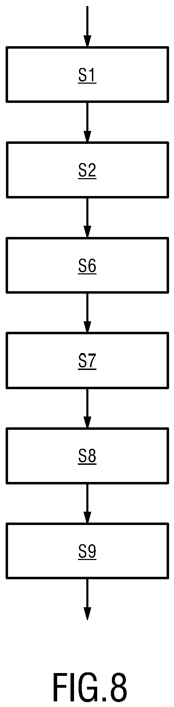

Fig. 8 shows a flow chart of a second embodiment of a method for generating a personalized action plan for a user according to the present invention.

In a first step S1 a text document comprising an original treatment plan is obtained. In a second step S2 keywords are extracted from said text document according to a predefined scheme. In particular, nouns, verbs and adjectives related to food, sports shopping and/or lifestyle may be extracted.

In step S6 categorization data are collected based on the keywords. In this embodiment, the categorization data are pre-categorized in a number of categories comprising "physiology target", "food type" and "action type". In fact, the categorization data may further be categorized in one or more sub-categories. For example, the category "physiology target" may comprise "macronutrient" as a sub-category. Likewise, the category "food type" may comprise "raw foods" as a sub-category. Furthermore, the sub-categories may comprise further sub-categories and so on. For example, the sub-category "micronutrient" may comprise the sub-category "proteins" and "vitamins" and the sub-category "raw foods" may comprise the sub-category "vegetable broccoli", for example. The keywords, are matched to one or more categorization data, and then all the categorization data in the same sub-categories with the categorization data matched with said keywords are collected.

In step S7 the collected categorization data are filled in action plan templates predefined in different classifications. Said classifications may refer to the sentence structures related to "what to eat?", "how to prepare?" and "what to do?" respectively. By filtering the filled action plan templates in step S8 according to the user data and prioritizing the filled action plan templates in step S9 according to the user data, there is generated a personalized action plan.. In this embodiment, user data may comprise the user's health status, behaviour preference and compliance with the personalized action plan. For example, for a pregnant woman suffering from vaginal bleeding action plan instructions related to exercises are filtered out. For users not eating pork, for example, action plan instructions related to pork are filtered out or their priority is lowered. Similarly, for users who are not able to provide their daily food as user data, the priority of action plan instructions related to daily food logs is lowered.

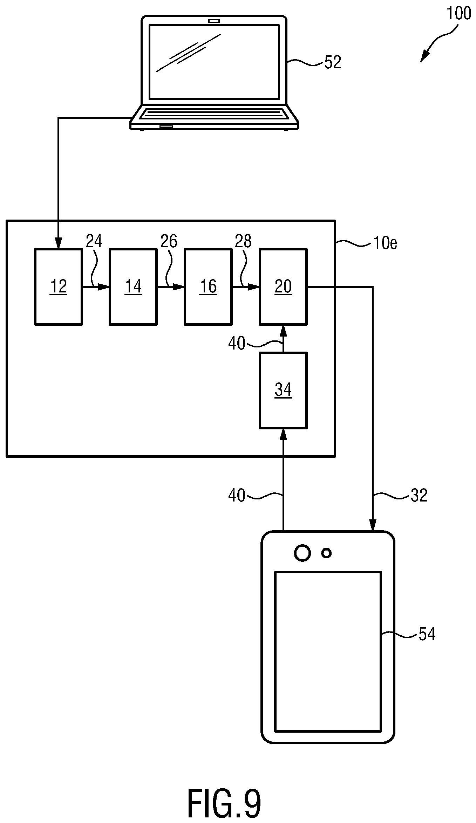

Fig. 9 shows a schematic diagram of an embodiment of a system 100 for generating a personalized action plan according to the present invention. The system 100 comprises a device 10e for generating a personalized action plan 32, text generation unit 52 implemented in a computer and a data output unit 54 implemented as a smartphone display.

In this embodiment, a doctor may write down the treatment plan for his/her patient on his/her computer, wherein said computer generates a corresponding text document. The text document comprising the treatment plan is then processed by the device 10e, which may be implemented in an external server, for example. In order to generate the personalized action plan 32 the action plan unit 20 takes into account user data 40 as obtained from the user data input 34. In this embodiment the user data input 34 is configured to obtain the user data 40 from the user's smartphone. The personalized action plan 32 is returned to the smartphone by the action plan unit 20, wherein the smartphone comprises a data output 54 configured as a display, wherein the action plan instructions may be displayed on the display.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A device (10) for generating a personalized action plan (32) for a user, the device (10) comprising:
- a keyword extraction unit (12) configured to obtain a text document (22) comprising a treatment plan and to extract a plurality of keywords (24) from said text document (22),
- a categorization data unit (16) configured to collect categorization data from a data set, based on the extracted plurality of keywords (24), wherein data in said data set are categorized into different categories, and
- an action plan unit (20) configured to generate the personalized action plan (32) by combining the collected categorization data (28) into action plan instructions.

2. The device (10) according to claim 1,
further comprising a user data input (34) configured to obtain user data (40), wherein the user data (40) comprises at least one of age, gender, weight, lifestyle data, fitness data, medical data, exercise behaviour data, dietary behaviour data.

3. The device (10) according to claims 1 or 2,
further comprising a matching unit (14) configured to match an extracted keyword (24) to categorization data, including the extracted keyword, in the data set, wherein the categorization data unit (16) is configured to collect the categorization data that are in a same category or subcategory as the matched categorization data.

4. The device (10) according to claims 2 to 3,
further comprising a filtering unit (36) configured to filter the action plan instructions, particularly according to the user data.

5. The device (10) according to any of the preceding claims,
further comprising a prioritizing unit (38) configured to prioritize the collected categorization data (28) and/or to prioritize the action plan instructions based on the user data (40).

6. The device (10) according to claims 2 to 5,
further comprising an effectiveness unit (42) configured to generate effectiveness data (46) based on the user data (40) and the personalized action plan (32), wherein the effectiveness data (46) comprises information about an effectiveness of the personalized action plan (42), wherein the action plan unit (20) is configured to adapt the personalized action plan (32) based on the effectiveness data.

7. The device (10) according to claims 2 to 6,
further comprising a compliance unit (44) configured to generate compliance data (48) based on the user data (40) and the personalized action plan (32), wherein the compliance data (48) comprises information about a compliance of the user with the personalized action plan (32), wherein the action plan unit (20) is configured to adapt the personalized action plan (32) based on the compliance data (48).

8. The device (10) according to any of claims 5 to 7,
wherein the prioritizing unit (38) is configured to prioritize the action plan instructions according to the effectiveness data (46) and/or the compliance data (48).

9. The device (10) according to claims 4 to 7,
wherein the filtering unit (36) is configured to filter the action plan instructions according to the effectiveness data (46) and/or the compliance data (48).

10. The device (10) according to any of the preceding claims,
wherein the categorization data are categorized into different categories comprising physiological target, food type, and action type.

11. The device (10) according to any of the preceding claims,
further comprising a text generation unit (52) configured to generate a text document from input data, wherein the input data comprise an image and/or a voice recording, and/or further comprising a data output unit (54) configured to output the personalized action plan (32), wherein the data output comprises any of a display, a printer and a loudspeaker, and/or further comprising a data input unit configured to generate the input data, wherein the data input unit comprises any of a camera, a scanner and a voice recorder.

12. A system (100) for generating a personalized action plan (32) for a user, the system comprising:
- a device (10) for generating a personalized action plan (32) for a user as claimed in claim 1, and
- a data input unit (50) configured to generate input data and/or a data output unit (54) configured to output the personalized action plan.

13. A method for generating a personalized action plan for a user, the method comprising:
- obtaining a text document (22) comprising a treatment plan and extracting a plurality of keywords (24) from said text document (22),
- collecting categorization data from a data set, based on the extracted plurality of keywords (24), wherein data in said data set are categorized into different categories, and
- generating the personalized action plan (32) by combining the collected categorization data (28) into action plan instructions.

14. The method according to claim 13,
further comprising matching a given keyword (24) to categorization data (26), including the given keyword, in the data set, and collecting categorization data that are in the same category or subcategory as the matched categorization data.

15. A computer program for generating a personalized action plan for a user comprising program code means for causing a computer to carry out the steps of the method as claimed in claim 13 or claim 14 when said computer program is carried out on the computer.
